Europäisches Patentamt

European Patent Office

Office européen des brevets

(19)

(11) Publication number: **0 204 510 B1**

## (12) EUROPEAN PATENT SPECIFICATION

(45) Date of publication of patent specification: **10.07.91**  (51) Int. Cl.⁵: **C12Q 1/68, //C07H21/00**

(21) Application number: **86304069.7**

(22) Date of filing: **29.05.86**

(54) Amplification of hybridization signals by employing complementary DNA strands.

(30) Priority: **31.05.85 US 739937**

(43) Date of publication of application:
**10.12.86 Bulletin 86/50**

(45) Publication of the grant of the patent:
**10.07.91 Bulletin 91/28**

(84) Designated Contracting States:
**AT BE CH DE FR GB IT LI LU NL SE**

(56) References cited:
**EP-A- 0 124 221**
**EP-A- 0 128 332**
**EP-A- 0 138 357**
**EP-A- 0 153 873**
**DE-A- 3 420 925**

(73) Proprietor: **AMOCO CORPORATION**
**200 East Randolph Drive**
**Chicago Illinois 60601(US)**

(72) Inventor: **Collins, Mark Leo**
**3409 W. Pierce Avenue**
**Chicago Illinois 60651(US)**

(74) Representative: **Lewin, John Harvey et al**
**ELKINGTON AND FIFE Beacon House 113**
**Kingsway**
**London WC2B 6PP(GB)**

Rank Xerox (UK) Business Services

## Description

BACKGROUND OF THE INVENTION

The present invention pertains to methods, reagent compositions and kits for enhancing the signal-generating capabilities of biological probes for use in the detection and quantitative analysis of target molecules In particular, the present invention relates to methods, reagent compositions and kits for performing deoxyribonucleic acid (DNA) or ribonucleic acid (RNA) hybridization assays in which the signal-generating capabilities of DNA or RNA probes are amplified or enhanced by the formation of a secondary network of labeled entities.

The following definitions are provided to facilitate an understanding of the present invention. The term "biological binding pair" as used in the present application refers to any pair of molecules which exhibit mutual affinity or binding capacity. For the purposes of the present application, the term "ligand" will refer to one molecule of the biological binding pair and the term "antiligand" or "receptor" will refer to the opposite molecule of the biological binding pair. For example, without limitation, embodiments of the present invention have application in nucleic acid hybridization assays where the biological binding pair includes two complementary strands of polynucleic acid. One of the strands is designated the ligand and the other strand is designated the antiligand. However, the biological binding pair may include antigens and antibodies, drugs and drug receptor sites, and enzymes and enzyme substrates to name a few.

The term "probe" refers to a member of a biological binding pair known to bind to a target ligand. As applied to nucleic acids, the term probe refers to a strand of nucleic acid having a base sequence complementary to a target strand. The term "label" refers to a molecular moiety capable of detection including, by way of example, without limitation, radioactive isotopes; enzymes; fluorescent, chemiluminescent, or precipitating agents; and dyes.

Genetic information is stored in living cells in the thread-like molecules of DNA or RNA. In vivo, the DNA molecule is a double helix, each strand of which is a chain of nucleotides. Each nucleotide is characterized by one of four bases: adenine (A), guanine (G), thymine (T), and cytosine (C). The bases are complementary in the sense that, due to the orientation of functional groups, certain base pairs attract and bond to each other through hydrogen bonding. Adenine in one strand of DNA pairs with thymine in an opposing complementary strand. Guanine in one strand of DNA pairs with cytosine in an opposing complementary strand.

The genetic code of a living organism is carried upon the DNA strand in the sequence of base pairs. DNA consists of covalently linked chains of deoxyribonucleotides and RNA consists of covalently linked chains of ribonucleotides. Each nucleic acid is linked by a phosphodiester bridge between the 5' hydroxyl group of the sugar of one nucleotide and the 3' hydroxyl group of the sugar of an adjacent nucleotide. Each linear strand of DNA or RNA has free 5' and 3' ends.

Nucleic acid hybridization assays are based on the tendency of two nucleic acid strands to pair and rewind at their complementary regions to form double helixes. The greater the extent of complementarity of their sequences, the greater is the tendency for a given pair of nucleic acid strands to associate and form duplexes.

Presently, nucleic acid hybridization assays are primarily used to detect and identify unique DNA or RNA base sequences or specific genes in a complete DNA or RNA molecule, mixtures of nucleic acids or mixtures of nucleic acid fragments. The identification of unique DNA or RNA sequences or specific genes, within DNA or RNA extracted from bacteria, may indicate the presence of genes for enzymes which convey antibiotic resistances. The identification of unique DNA sequences or specific genes, within DNA extracted from human or animal tissue, indicates the presence of genes which predispose the human or animal to cancer or genetic diseases such as sickle-cell anemia.

Thus, nucleic acid hybridization assays have great potential in the diagnosis or detection of diseases including genetic or chromosomal abnormalities, antibiotic resistance in infectious diseases and tissue compatibility. Further potential exists in agriculture and food processing where nucleic acid hybridization assays may be used to detect plant pathogens or toxin-producing bacteria.

In a typical polynucleotide hybridization test, two DNA specimens are mixed and heated beyond the dissociation temperature of both and then cooled slowly. If the two DNA specimens possess complementary base sequences, they will associate to form hybrid duplexes. The extent to which such hybrids form is easily determined if one of the DNA specimens is first labeled with a radioactive isotope of phosphorus ($^{32}P$) and the other specimen is immobilized on a solid support. After allowing the two DNA specimens to hybridize, the unhybridized DNA strands can be separated from the immobilized DNA strands by simple washing of the surface to which the DNA strand is immobilized. The surface can then be tested for radioactivity exhibited by the radioactive label of the opposing DNA strand.

One of the most widely used procedures is known as the Southern blot filter hybridization method or simply the Southern procedure (Southern, E., J. Mol. Biol., 98, 503, 1975). The Southern procedure is used to identify target DNA sequences. The procedure is generally carried out by subjecting sample DNA, potentially containing the target sequence isolated from an organism, to restriction endonuclease digestion to form DNA fragments. The sample DNA fragments are then electrophoresed on a gel such as agarose or polyacrylamide to sort the sample fragments by length. Each group of fragments can be tested for the presence of the target sequence. The DNA is denatured inside the gel to enable transfer to nitrocellulose sheets. The gel containing the sample DNA fragments is placed in contact (blotted) with a nitrocellulose filter sheet or diazotized paper. The sample DNA fragments are transferred and become bound to the nitrocellulose sheet or diazotized paper. The nitrocellulose sheet containing the sample DNA fragments is then heated to approximately 85°C to immobilize the DNA. The nitrocellulose sheet is then treated with a solution containing a denatured radiolabeled DNA probe. The radiolabeled DNA probe includes a strand of DNA having a base sequence complementary to the target sequence and having a radioactive moiety which can be detected. Hybridization between the probe and sample DNA fragments is allowed to take place. During the hybridization p rocess, the sample DNA is allowed to recombine with labeled DNA probe and again form double-stranded structures. The hybridization process is very specific. The labeled DNA probe will not recombine with sample DNA if the two DNA entities do not share complementary base pair organization. Hybridization can take from 3 to 48 hours, depending on given conditions. Unhybridized DNA probe is subsequently washed away. The nitrocellulose sheet is then placed on a sheet of X-ray film and allowed to expose. The X-ray film is developed with the exposed areas on the film identifying DNA fragments which have hybridized to the DNA probe and therefore have the base pair sequence of interest.

The use of nucleic acid hybridization assays has been hampered in part by the rather long exposure times required to visualize bands on X-ray film. A typical Southern procedure may require 1-7 days for exposure alone. Further, many of the present techniques require radioactive isotopes as labeling agents. The use of radioactive labeling agents requires special laboratory procedures and licenses. Nonradioactive assays, utilizing enzymatic, fluorescent or chemiluminescent label moieties, have not had the sensitivity or the specificity necessary to be considered reliable hybridization assays.

In an effort to increase the sensitivity of nonradioactive hybridization assays, the Enzo Biochem Company (ENZO) located in New York has marketed a DNA assay system under the trademark BIO-BRIDGE. The ENZO system uses an enzyme, terminal transferase, to build "tails" of thymidine (poly T) on the terminal 3' hydroxyl position of the deoxyribose sugar backbone of DNA fragments for use as a poly T-tailed probe. The poly T-tailed probe is allowed to hybridize to immobilized target DNA. Unhybridized poly T-tailed probe is washed or otherwise removed. Next, a plurality of labeled homopolymer strands of adenosine (poly A), each incorporating at least one biotin molecule, is hybridized to the poly T tail of the probe. Biotin is a nonradioactive label moiety. Biotin is detectable by the interaction of biotin with biotin-binding proteins which in turn are coupled to fluorescent dyes or color-producing enzymes. Unhybridized poly A strands are washed away or removed. Detection of the biotin on the label is indicative o f the presence of target DNA. The signal-enhancing capabilities of the ENZO system appear to be limited.

In particular, there are practical limits to the length of tail segments incorporated on probe segments. As the length of the tail segment increases, hybridization efficiency and sensitivity decrease. As the length of the tail portion is limited, so is the ability to incorporate label moieties into the tail or to hybridize additional polynucleotide-labeled segments to the tail.

## SUMMARY OF THE INVENTION

It is an object of the present invention to provide methods, reagent compositions and kits for performing assays for molecules of interest which are members of a biological binding pair. Other objects will be presented hereinafter.

Briefly, an embodiment of the invention includes a method for assaying samples for a target member of a biological binding pair. The method includes contacting sample which may contain target ligand of a biological binding pair with reagent under binding conditions. The reagent includes receptor probe, amplifier strand and label strand. The receptor probe is capable of specifically binding to the target ligand under binding conditions. The receptor probe further includes a polynucleotide tail portion capable of binding to the amplifier strand. The amplifier strand includes a polynucleotide strand capable of binding to the tail portion and to at least one of the label strands. The label strand includes a polynucleotide strand having a label moiety capable of detection. Unbound reagent is removed from the sample and the sample monitored for the presence of the label moiety. The present invention allows a single target molecule to initiate a series of hybridizations forming a network of polynucleotide amplifie r and label strands. The network enhances the signal-generating capacity of the single target molecule.

Preferably, the tail portion of the receptor probe includes a homopolymer of DNA or RNA such as poly A or poly T. The amplifier strand is preferably a homopolymer of DNA or RNA which is complementary to the tail portion. Thus, if the tail portion of the receptor probe is a poly A homopolymer of DNA or RNA, one amplifier strand would preferably include a homopolymer of poly T. Similarly, if the tail portion of the receptor probe is a poly T homopolymer of DNA or RNA, the amplifier strand would preferably include a homopolymer of poly A.

Preferably, the label strand is a DNA or RNA homopolymer complementary to the amplifier strand. Further, the label strand includes a label moiety which is capable of detection. The label moiety may include, by way of example without limitation, a radioisotope, nonradioactive entities, such as enzymes, fluorescent, chemiluminescent, or precipitating agents or dyes. Those skilled in the art will recognize that other reagent entities, such as the amplifier strand, the tail portion of the receptor probe, and the receptor probe itself may also carry label moieties.

One embodiment of the present invention includes a method for assaying sample for single-stranded polynucleotide target segments. The method includes contacting sample potentially containing single-stranded polynucleotide target segments with reagent polynucleotide strands under hybridization conditions. The reagent polynucleotide strands include polynucleotide probe strand, polynucleotide amplifying strand, and polynucleotide label strand. The probe strand includes base sequences complementary to a representative portion of the target segment. The probe strand also includes a polynucleotide tail portion capable of binding to the amplifier strand. The amplifier strand is capable of binding to the label strand. The label strand includes a label moiety which is detectable. After hybridization has taken place, unhybridized reagent strands are removed from the sample and the sample is monitored for the presence of the label moiety. The presence of the label moiety indicates the presence of the target single-str anded polynucleotide segment in the sample. The magnitude of the label response is an indication of the concentration of the target single-stranded polynucleotide segment in the sample.

A preferred embodiment includes an amplifier strand capable of binding to a plurality of label strands allowing for the signal-generating capability of the target polynucleotide segment and polynucleotide probe strand.

A further embodiment of the present invention includes the additional step of contacting the sample with a second set of amplifier strand and label strand. The second amplifier strand is capable of binding to the preceding amplifier strand and the label strand is capable of binding to the second amplifier strand. The additional step may be repeated to build a network of amplifier strands and label strands until acceptable signal levels are achieved.

A further embodiment of the present invention includes a reagent composition for use with a probe strand having a polynucleotide tail portion. The reagent composition comprises a polynucleotide amplifier strand and a polynucleotide label strand. The amplifier strand is complementary to a representative segment of the tail portion of the probe strand. The polynucleotide label strand is complementary to a representative segment of the polynucleotide amplifier strand. The label strand includes a label moiety capable of detection.

A further embodiment includes a reagent composition including a polynucleotide probe strand as well as an amplifier strand and a label strand. Again, the polynucleotide probe strand has a tail portion complementary to a representative segment of the amplifier strand.

An embodiment further includes a reagent composition wherein reagent strands are hybridized to each other. The reagent strands are placed in contact with sample single-stranded polynucleotide segments under hybridization conditions to form a network of reagent strands.

A further embodiment of the present invention includes a kit for assaying target single-stranded polynucleotide segments in a sample. The kit includes reagent single-stranded polynucleotide probe strands which are complementary to a representative portion of a target single-stranded polynucleotide segment. Each reagent probe strand includes a polynucleotide tail portion capable of binding to a complementary reagent polynucleotide amplifier strand. The kit further includes a reagent polynucleotide amplifier strand and a reagent polynucleotide label strand. The label strand is complementary to the amplifier strand and includes a label moiety capable of detection. Under hybridization conditions, the reagent probe strand hybridizes to the sample target segment. The amplifier strand hybridizes to the tail portion of the reagent probe strand, and the reagent label strand hybridizes to the amplifier strand to form a network of reagent strands hybridized to the target segment.

A further embodiment includes a kit having a second amplifier strand and a second label strand. The second amplifier strand is capable of binding to the first amplifier strand. The second label strand is capable of binding to the second amplifier strand. The application of the first and second amplifier strand and label strands can be repeated to build up a network of amplifier and label strands and increasing the detectable signal.

The present method, reagent composition, and kit provide for improved specific activities which approach five times the activity of conventional assay techniques and kits. The high specific activity of the reagent network formed during the procedure minimizes exposure time.

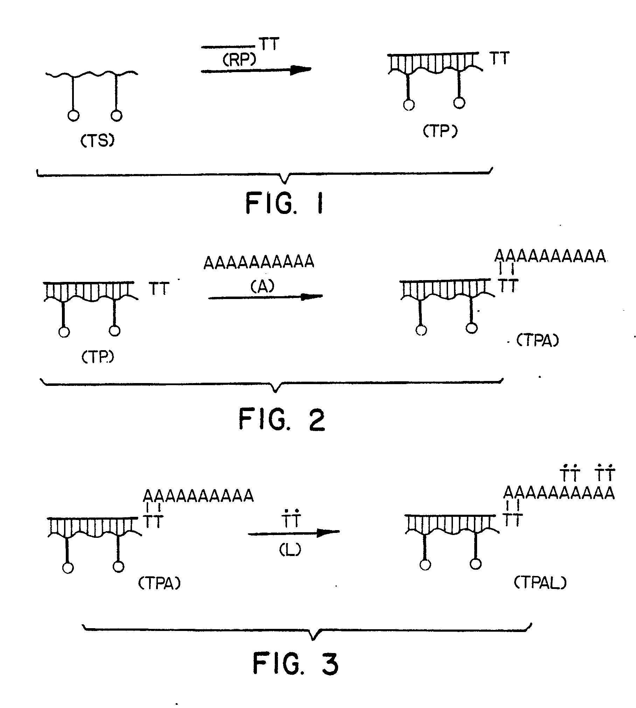

Turning now to the drawing, which by illustration depicts preferred embodiments, a method of procedure, with necessary reagent compositions, is illustrated in schematic form for an assay for a target polynucleotide strand. Figure 1 depicts a target strand (TS) immobilized on a solid substrate such as nitrocellulose paper. In conventional assay techniques, more than one target strand would be immobilized; however, for simplicity to further an understanding of the invention, the illustrations depict only a single target strand. The target strand is brought into contact with a reagent probe (RP) which has a base sequence complementary to a representative portion of the target strand. Under hybridization conditions, the target strand and the probe strand form a target-probe duplex (TP) immobilized to the support.

The probe strand has a tail portion including a homopolymer of poly T which is capable of binding to an amplifier strand including a complementary base sequence, a homopolymer of poly A. The tail portion and amplifier strands are illustrated with arbitrary numbers of nucleotides chosen for simplicity to facilitate an understanding of the invention. It will also be recognized that the illustrated binding sites of the homopolymers are arbitrary.

Turning now to Fig. 2, the amplifier strand (A) is brought into contact with the tail portion of the polynucleotide probe under hybridization conditions. The tail portion and the amplifier strand quickly form a target-probe-amplifier complex (TPA).

The tail and amplifier strand allow for multiple binding sites for a label strand (L) including a base sequence complementary to the amplifier strand, a homopolymer of poly T. The label strand (L) includes label moieties capable of detection generally designated with dots above the capital letter representing the nucleotide. Under hybridization conditions, substoichiometic quantities of the label strand (L) bind to portions of the amplifier strand (A) to form target-probe-amplifier-label complex (TPAL) as illustrated in Fig. 3. Unbound reagent probe strand, amplifier strand and label strand can be washed and the sample monitored for the presence of label. The presence of label is indicative of the presence of the target strand.

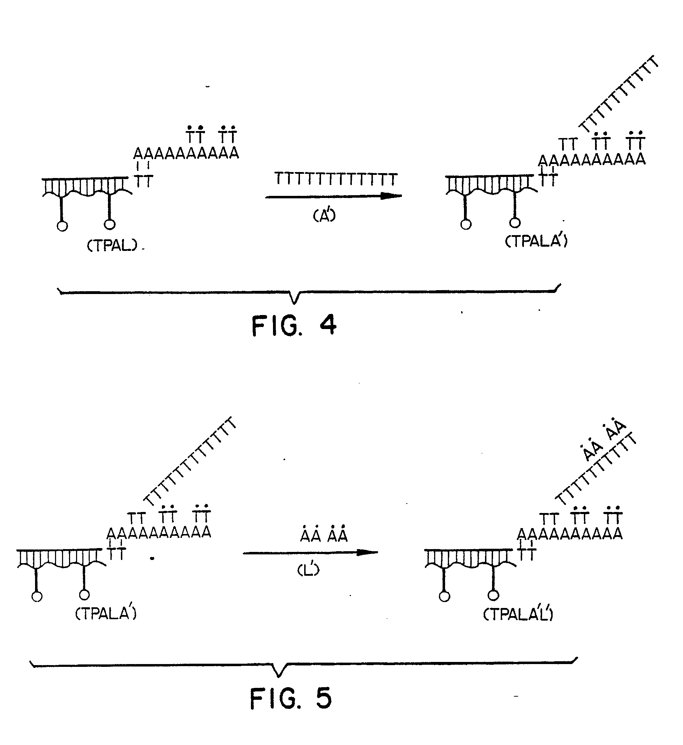

For some applications, particularly those involving nonradioactive label moieties, it is desirable to further amplify or enhance the signal-generating abilities of the target-probe-amplifier-complex (TPAL). Thus, the first amplifier strand (A) and label strand (L) are brought into contact with a second amplifier strand (A') such as a homopolymer of T capable of binding to unhybridized portions of the first amplifier strand. Under hybridization conditions, the second amplifier strand (A') forms a duplex with free areas along the first amplifier strand to form the structure identified by the letters TPALA' illustrated in Fig. 4.

A second label strand (L') capable of binding to the second amplifier strand (A') is brought into contact with the second amplifier strand (A') under hybridization conditions. The second label strand (L') quickly forms duplexes with portions of the second amplifier strand (A') to form the complex designated TPALA'L' as best seen in Fig. 5.

Preferably, after each hybridization step, unhybridized reagent strands are removed from the sample. After the final hybridization step and final wash, the sample is monitored for the presence of label.

It will be recognized that the second amplifier strand and second label strand can be followed with additional amplifier and label strand combinations. Further, those skilled in the art will recognize that the label strand and amplifier strand combination can be brought into contact with target and reagent strands as a single entity, reducing the number of steps.

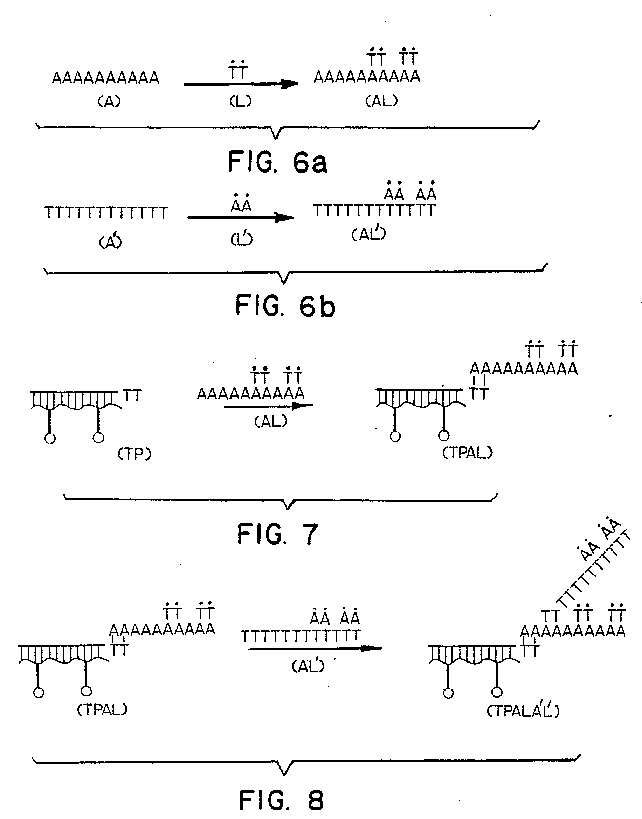

Thus, in Fig. 6a, a first amplifier-label complex (AL) is formed by contacting the first amplifier strand (A) with a substoichiometic amount of a label strand (L). Similarly, a second amplifier-label complex (AL') is formed by contacting the second amplifier strand (A') with a substoichiometic amount of a second label strand (L') as illustrated in Fig. 6b.

The target-probe duplex of Fig. 1 is brought into contact under hybridization conditions with the first amplifier-label complex (AL) to form a complex designated TPAL as illustrated in Fig. 7. Next, the second amplifier-label complex (AL') is brought into contact with the complex TPAL under hybridization conditions to form a larger network of amplifier label strands designated as TPALA'L' illustrated in Fig. 8.

Again, it is preferred after each hybridization step, unhybridized reagent strands are removed from the sample. After the final hybridization step and final wash, the sample is monitored for the presence of label.

The hybridization of homopolymer reagent strands occurs quickly. The base pairs do not need to be positioned to find a complementary arrangement. After one base pair is in position for mutual attraction, the others are readily aligned.

The rate limiting step, the hybridization of the target strand to the probe strand, is not impeded by large label moieties on the tail portion or by an extremely large tail portion on the probe strand. Further hybridization steps between homopolymer strands proceed quickly allowing the assay procedure to be performed in a relatively short time.

In embodiments of the present invention utilizing radioactive labels, the present invention provides a specific activity much greater than conventional techniques. Film exposed to the radioactivity react faster to the stronger signals, decreasing exposure time. Alternatively, lower concentrations of target strands can be detected.

In embodiments of the present invention utilizing nonradioactive enzymatic, chemiluminescent or fluorescent label moieties, the label moieties and cofactors for signal-generating reaction may be affixed to reagent polynucleotide strands to bring label moieties and cofactors in close mutual proximity. The term "cofactor" is used broadly herein to include all molecular species which may participate in reactions which produce a detectable response. For example, chemiluminescent and fluorescent moieties are combined to provide energy transfer or chemiluminescent label moieties are combined with reactants to provide light-emitting reactions. The cofactors and other label moieties are localized on different reagent strands which are brought together upon the formation of a network of strands to increase the possibility of detectable reactions taking place.

The present invention is further illustrated and described in the following experimental examples which exemplify features of preferred embodiments.

EXAMPLE

In the present example all labeled nucleotides were obtained from New England Nuclear. The plasmid pBR322 was obtained from Bethesda Research Labs (BRL). The plasmid pBR322 was restricted with the enzyme Hha I, obtained from BRL, creating multiple restriction fragments of the plasmid. The enzyme terminal deoxynucleotidyl transferase (tdt) was obtained from Life Sciences, Inc., St. Petersburg, Florida. Bovine serum albumin was obtained from Bethesda Research Labs of Gaithersburg, Md. Polydextran used in the example chromatography is marketed under the trademark SEPHADEX G-25 by Pharmacia PL Biochemicals, Inc. of Piscataway, N.J. Nitrocellulose filters and blot apparatus used in the example are marketed under the trade designation BA85 for filters and Minifold I for apparatus by Schleicher and Schuell. All other reagents were of analytical grade.

In the present examples, SDS buffer includes 0.1 M sodium chloride, 10 mM tris(hydroxymethyl) aminomethane hydrochloride (pH 7.4), 5 mM ethylene diamine tetraacetate (EDTA) and 0.5% sodium dodecyl sulfate (SDS). The equal volume mixture of phenol and chloroform contained 1% isoamyl alcohol. The chloroform used in the examples also contained 1% isoamyl alcohol. The lx sodium chloride/sodium citrate buffer (SSC) of the present example included 0.15 sodium chloride and 0.015 sodium citrate (pH 7.3).

Terminal deoxynucleotidyl transferase was used to build tails to the restriction fragments of pBR322 to form a reagent polynucleotide probe strand having a homopolymer tail. In a reaction volume of 60 microliters, 100 ng of the restriction fragments were incubated for 60 minutes at 37°C in 100 mM potassium cacodylate (pH 7.0), 1 mM cobalt chloride, 100 mM beta-mercaptoethanol, 25 microcuries of tritium-labeled thymidine triphosphate (dTTP) (100 Ci/mmole), 0.1 mg/ml bovine serum albumin and 100 units of terminal deoxynucleotidyl transferase. Terminal deoxynucleotidyl transferase adds in a sequential manner free nucleotides to the 3' end of a strand of DNA. Thus, reagent probe strands were prepared having a probe portion complementary to portions of plasmid pBR322, by virtue of their origin, and a tail portion of poly-deoxythymidine (poly T) which is capable of binding to a complementary strand of poly-deoxyadenosine (poly A). An average of 85 deoxythymidine residues were added to the reaction fragments as determined in calculations set forth later in the application.

Reagent amplifier strands, polynucleotide strands complementary to tail portions on reagent polynucleotide probe strands, can be obtained from several companies which specialize in biologicals, such as Life Sciences, Inc. or synthesized with the enzyme terminal deoxynucleotidyl transferase as suggested by the protocol which follows pertaining to the synthesis of reagent labeled strands. The reagent amplifier strand of the present example included a poly A homopolymer having an average length of 4000 nucleotides.

Reagent label strands were synthesized utilizing terminal deoxynucleotidyl transferase. Using terminal deoxynucleotidyl transferase, tritium-labeled deoxythymidine triphosphate ($^3$H-dTTP) was enzymatically added to homopolymer strands of poly T of sixteen nucleotide length $(dpT)_{16}$ to form homopolymer strands of poly T of 65 nucleotide length $(dpT)_{65}$ having a radioactive label moiety.

Thus, in a reaction volume of 60 microliters, 10 ng of homopolymer strands of poly T of sixteen nucleotide length were incubated for 60 minutes at 37°C in 100 mM potassium cacodylate (pH 7.0), 1 mM cobalt chloride, 1 mM beta-mercaptoethanol, 25 microcuries of tritium-labeled deoxythymidine triphosphate (100 Ci/mmole), 100 micrograms/ml bovine serum albumin, and 100 units of terminal deoxynucleotidyl transferase.

The resultant reagent label strands were hybridized to reagent amplifier strands to form a reagent amplifier-label complex which was saved until used. Thus, 33 ng of radioactive-labeled polydeoxythymidine (65 nucleotides in length) were incubated with 50 ng of polydeoxyadenosine (4,000 nucleotides in length) in 2X sodium chloride-sodium citrate buffer at 55°C for 60 minutes.

The reagent probe strands and the reagent amplifierlabel complexes were purified using standard procedures. Thus, 10 volumes of SDS buffer were added to the reactant vessels containing the respective reagent probe strands and amplifier-label complexes. The respective reagent probe strands and amplifier-label complexes were extracted twice with an equal volume mixture of phenol and chloroform and once with chloroform. The organic residues and residual nucleotides were removed from the respective reagent probe strands and amplifier-label complexes by two successive spins through 10-15 volumes of SEPHADEX G-25 brand polydextran packed columns equilibrated with 10 mM of EDTA (pH 7.0). The polydextran was heated to 37°C and degassed prior to its use in spun column chromatography.

After pelleting the SEPHADEX G-25 brand polydextran fines, SDS was added in quantities sufficient to make a 0.2% concentration in the supernatant liquid containing the respective probes. The supernatant liquid was stored at 4°C until ready for use. Typically, the supernatant liquid contained 50% of the theoretical yield of respective reagent probe strands and amplifier-label complex. As described herein, the tail of the reagent probe strand bears radioactive labels. However, it will be recognized that the label incorporated within the tail is not necessary for the operation of the reagent amplifier strand or the reagent label strand. However, the label does permit the calculation of the average tail length of the target probe.

The average tail length, L, of both the probe strand and the label strand, was calculated as follows:

$$L = (TCA\ cpm \times 325 \times m)/(c \times 2.2 \times 10^3 \times Sa \times M).$$

In the above equation, TCA cpm equals the total radioactivity in the sample that can be precipitated by the addition of trichloroacetic acid. In our examples, the probe strand had a TCA cpm of $6 \times 10^6$, and the label strand had a TCA cpm of $3 \times 10^6$.

The numeral 325 equals the approximate average mass of a sodium deoxyribonucleotide measured in daltons. The number of nucleotides per strand is represented by m. In our examples, the probe strand had an average number of nucleotides of 141, and the label strand had an average number of nucleotides of 16. In the case of the label strand the value is known due to the use of synthetic oligonucleotide starting materials. In the case of the probe strand, the average number of nucleotides is calculated from the known number of base pairs in the pBR322 plasmid, 4362 and known Hha I sites, 31.

Continuing, c represents the counting efficiency of the nucleotide. The scintillation fluid, Scintiverse II (Fischer Scientific) revealed a 35% counting efficiency for the tritium-labeled probes. The number $2.2 \times 10^3$ represents the number of disintegrations per minute in one nanocurie of the isotope. The mass (M) is measured in picograms (pg). The respective masses of the label strand and probe strand were estimated in picograms by measuring the optical density of the concentrated stocks. The calculations assumed that 1 $OD_{260}$ was roughly equivalent to 40 μg/ml of target probe. In our examples, the mass of the probe strands was 100 ng, and the mass of the label probe was 10 ng.

In the calculations, the value for specific activity was measured in disintegrations per minute per femtogram of probe (dpm/fg) added to the reaction mixture. In this method, the mass of the tail is not considered to be part of the mass of the reagent probe. The tail is considered to be label only. This method was used since it is convenient in estimating the apparent hybridization efficiency (E). In our examples, the reagent probe strand had a specific activity of 0.4 dpm/fg, and the label strand had a specific activity of 0.5 dpm/fg. The apparent hybridization efficiency can be calculated as set forth in the equation below:

$$E = (dpm\ hybridized/Sa)/(fg\ of\ complementary\ sequence).$$

The value for the hybridization efficiency is considered apparent because the specific activity of the probes actually hybridized may well be lower than that of the probe as a whole. In using this method for determining the hybridization efficiency, the specific activity of the reagent probe is greater than that of the label (dpm/fg of label) whenever the average tail length, L, is greater than the original length of the probe.

In completing the calculations, it was determined that the reagent probe carried a tail of tritium-labeled dTMP of 85 residues. It was further determined that the reagent label strand had an average length of 65 deoxythymine residues. The apparent hybridization efficiency of the probe strand to the target strand was 1%, and the apparent hybridization efficiency of the label strand to amplifier strand was 100%.

The target DNA, plasmid pBR322, was immobilized by heat-denaturing 1 ng of the plasmid at 100°C in 0.1 M sodium hydroxide for 5 minutes. The plasmid-sodium hydroxide mixture was then added to 25 volumes of ice-cold 2 M sodium chloride and slowly filtered through nitrocellulose filters (BA85) in a dot blot apparatus. The dots, containing the denatured target DNA, were neutralized in 10x sodium chloride/sodium citrate buffer (SSC), dried under an infrared lamp and baked for at least two hours at 85°C under reduced pressure. The dots affixed to nitrocellulose filter paper were prehybridized twice.

The prehybridization process saturates remaining DNA binding sites on the nitrocellulose filter. Reagent polynucleotide strands will therefore not bind to the nitrocellulose filter paper but will only affix to immobilized DNA of complementary base sequence. The first prehybridization step was at 65°C for at least two hours in prehybridization buffer including 2x SSC, 0.5% SDS, 0.2% polyvinylpyrrolidone, 0.2% ficoll, and 0.1 mg/ml proteinase K (Boehringer Mannheim). The second prehybridization step was at 10-20°C below the disassociation temperature of the reagent probe strand to the target strand as determined below, for at least two hours in the above prehybridization buffer.

Reagent probe strands with tail portions were hybridized to the plasmid affixed to the nitrocellulose filter at 65°C, which is 10-20°C below the estimated disassociation temperatures for Hha I fragments of pBR322 plasmid. Hybridization took place in the above prehybridization buffer.

Some of the blots were blotted dry on Whatman filters, dried under an infrared lamp and counted in a scintillation counter. Reagent probe strands hybridized to the plasmid DNA demonstrated a signal strength ranging from 950 to 1,600 cpm (above that of a filter without target DNA).

Blots that were not counted were subjected to a further hybridization step with the amplifier-label complex. The amplifier-label complex was allowed to hybridize to the tail portion of the reagent probe at a temperature of 55°C for 30 minutes in 2x SSC buffer solution containing 0.5% SDS. The amplifier-label complex was the equivalent of 33 ng of polydeoxythymidine of 65 base length and 50 ng of polydeoxyadenosine.

Blots hybridized to the reagent amplifier strands and reagent label strands were washed with several changes of 2x SSC buffer including 0.5% SDS at 55°C for 10 minutes.

The blots were blotted dry on Whatman filters, dried under an infrared lamp and counted in a scintillation counter. The scintillation count indicated that the blots subjected to the amplifier-label complex had a specific activity of 7,500 cpm (above that of a filter without target). Blots subjected to the probe strand had an activity of no more than 1,600 cpm. Blots previously exhibiting a specific activity of 950 cpm when subjected to the probe strand now demonstrated a specific activity of 3,800 cpm when subjected to the amplifier-label complex. Thus, hybridization with the amplifier-label complex enhanced the signal between 4 and 4.5 times that of a simple radioactive-tailed probe. Each additional application of an amplifier-label complex would enchance the signal by a factor of two.

Thus, the present invention features means for amplifying weak specific activities of analytical probes without elaborate organic chemistry. Further, although the present example features radioactive labeling means, embodiments of the present invention also can be used for nonradioactive labeling techniques where low signals have been a significant problem. For example, the label strand may include chemiluminescent agents such as microperoxidase, iron porphyrin derivatives, bacterial luciferase, firefly luciferase, flavin mononucleotide, adenine triphosphate, and nicotinamide adenine dinucleotide and its derivatives. Suitable fluorescent light labels include a variety of fluorescent nucleotides such as ethenoadenosine nucleotides or etheno-cytidine nucleotides, or functionalized nucleotides such as amino-hexane adenosine nucleotides or mercurinucleotides.

Further, due to the improved sensitivity, hybridization results can be achieved much faster because a detectable signal is obtained in a shorter time. The rate-limiting step, namely the initial hybridization of the target strand to probe strand is not impaired by large label moieties or long chains of oligonucleotides. The amplifying or enhancing steps, using homopolymers, proceeds quickly providing for rapid results.

Thus, while preferred embodiments have been illustrated and described, it is understood that the present invention is capable of variation and modification and, therefore, should not be limited to the precise details set forth, but should include such changes and alterations that fall within the purview of the following claims.

## Claims

1. A method for assaying sample for a target ligand of a biological pair comprising the steps of:

contacting sample with reagent under binding conditions wherein said reagent includes reagent probe, amplifier strand, and label strand, said reagent probe capable of binding to said target ligand and having a polynucleotide tail portion, said amplifier strand including a polynucleotide strand capable of binding to said tail portion and to a plurality of said label strands, and said label strand including a polynucleotide strand having a label moiety capable of detection, said reagent for forming a network of polynucleotide strands bound to said target ligand;

removing unbound reagent from sample; and monitoring said sample for said label moiety.

2. The method of Claim 1 wherein said reagent includes a second amplifier strand and a second label strand, said second amplifier strand capable of binding to said first amplifier strand and said second label strand capable of binding to said second amplifier strand and including a label moiety, said method further comprising the additional step prior to monitoring and after contacting said sample with reagent the sample probe amplifier strand and label strand, comprising contacting said sample said second amplifier strand and said label strand under binding conditions to form a network of polynucleotide strands.

3. The method of Claim 1 or 2 wherein said amplifier strand is capable of binding to a plurality of label strands.

4. The method of any of Claims 1 to 3 wherein at least one of said amplifier strand and probe strand includes label moieties.

5. The method of any of Claims 1 to 4 wherein said regent probe includes a polynucleotide probe portion complementary to said target ligand.

6. The method of any of Claims 1 to 5 wherein said tail portion of said reagent probe, said amplifier strand, and said label strand include homopolymers.

7. The method of any of Claims 1 to 6 wherein said reagent sample is first contacted with reagent probe, said target ligand is allowed to bind to said reagent probe to form a ligand-receptor complex, followed by contacting said ligand-reagent complex with a reagent amplifier strand and reagent label strand wherein said amplifier strand and label strand are bound to each other to form an amplifier label complex.

8. The method of any of Claims 1 to 7 wherein said label moiety is nonradioactive.

9. A reagent composition for enhancing the signal-generating capabilities of a reagent probe having a polynucleotide tail portion comprising: a polynucleotide amplifier strand and a plurality of label strands bound thereto, said amplifier strand capable of binding to a reagent probe, said label having a label moiety capable of detection, said amplifier strand for binding to said reagent probe and said label strand binding to said amplifier strand forming a network of polynucleotide strands capable of detection.

10. The reagent composition of Claim 9 wherein at least one of said amplifier strand and said probe strand includes label moieties.

11. The reagent composition of Claim 9 or 10 wherein said amplifier strand and label strand are complementary homopolymers.

12. A kit for assaying sample for target ligand of a biological pair comprising:

reagent including reagent probe, amplifier strand and label strand, said reagent probe capable of binding to said target ligand and having a polynucleotide tail portion, said amplifier strand including a polynucleotide strand capable of binding to said polynucleotide tail of said reagent probe, and, said label strand capable of binding to said amplifier strand and having a label moiety capable of detecting said reagent for forming a network of polynucleotide strands capable of detection bound to said target ligand.

13. The kit of Claim 12 further comprising a second amplifier strand and a second label strand, said second amplifier strand capable of binding to said first amplifier strand and said second label strand capable of binding to said second amplifier strand.

14. The kit of Claim 12 or 13 wherein said reagent probe includes a polynucleotide probe portion complementary to said target ligand.

15. The kit of any of Claims 12 to 14 wherein said tail portion of said reagent probe, said amplifier strand and said label strand include homopolymers.

16. The kit of any of Claims 12 to 15 wherein said amplifier strand and said label strand are bound to each other in an amplifier-label complex.

17. The kit of any of Claims 12 to 16 wherein said label moiety is nonradioactive.

## Revendications

1. Un procédé pour la détermination, dans un échantillon, d'un ligand cible d'une paire biologique comprenant les étapes de :

contact de l'échantillon avec un réactif dans des conditions de liaison, ledit réactif comprenant une sonde réactive, un brin amplificateur et un brin marqueur, ladite sonde réactive étant capable de se fixer audit ligand cible et ayant une portion de queue polynucléotidique, ledit brin amplificateur comprenant un brin polynucléotidique capable de se lier à ladite portion de queue et à plusieurs desdits brins marqueurs, et ledit brin marqueur comprenant un brin polynucléotidique ayant un fragment marqueur susceptible de détection, ledit réactif formant un réseau de brins polynucléotidiques fixé audit ligand cible ;

l'élimination du réactif non fixé de l'échantillon ; et

l'analyse dudit échantillon pour déterminer ledit fragment marqueur.

2. Le procédé de la revendication 1, dans lequel ledit réactif comprend un second brin amplificateur et un second brin marqueur, ledit second brin amplificateur étant capable de se fixer audit premier brin amplificateur et ledit second brin marqueur étant capable de se fixer audit second brin amplificateur et comprenant un fragment marqueur, ledit procédé comprenant de plus l'étape additionnelle, avant l'analyse et après le contact dudit échantillon avec la sonde d'échantillon, le brin amplificateur et le brin marqueur réactifs, consistant en le contact dudit échantillon, dudit second brin amplificateur et dudit second brin marqueur dans des conditions de liaison pour former un réseau de brins polynucléotidiques.

3. Le procédé de la revendication 1 ou 2, dans lequel ledit brin amplificateur est capable de liaison à plusieurs brins marqueurs.

4. Le procédé de l'une quelconque des revendications 1 à 3, dans lequel au moins un dudit brin amplificateur et dudit brin sonde comprend des fragments marqueurs.

5. Le procédé de l'une quelconque des revendications 1 à 4, dans lequel ladite sonde réactive comprend une portion de sonde polynucléotidique complémentaire dudit ligand cible.

6. Le procédé de l'une quelconque des revendications 1 à 5, dans lequel ladite portion de queue de ladite sonde réactive, ledit brin amplificateur et ledit brin marqueur comprennent des homopolymères.

7. Le procédé de l'une quelconque des revendications 1 à 6, dans lequel ledit échantillon réactif est tout d'abord mis en contact avec la sonde réactive, on laisse ledit ligand cible se fixer à ladite sonde réactive pour former un complexe ligand-récepteur, puis on met ledit complexe ligand-récepteur en contact avec un brin amplificateur réactif et un brin marqueur réactif, ledit brin amplificateur et ledit brin marqueur étant liés entre eux pour former un complexe amplificateur-marqueur.

8. Le procédé de l'une quelconque des revendications 1 à 7, dans lequel ledit fragment marqueur est non radioactif.

9. Une composition réactive pour accroître les capacités de production d'un signal d'une sonde réactive ayant une portion de queue polynucléotidique, comprenant : un brin amplificateur polynucléotidique et plusieurs brins marqueurs qui lui sont liés, ledit brin amplificateur étant capable de liaison à une sonde réactive, ledit marqueur ayant un fragment marqueur susceptible de détection, ledit brin amplificateur se liant à ladite sonde réactive et ledit brin marqueur se liant audit brin amplificateur pour former un réseau de brins polynucléotidiques susceptible de détection.

10. La composition réactive de la revendication 9, dans laquelle au moins un dudit brin amplificateur et dudit brin sonde comprend des fragments marqueurs.

11. La composition réactive de la revendication 9 ou 10, dans laquelle ledit brin amplificateur et ledit brin marqueur sont des homopolymères complémentaires.

12. Un nécessaire pour déterminer un ligand cible d'une paire biologique dans un échantillon comprenant :

un réactif comprenant une sonde réactive, un brin amplificateur et un brin marqueur, ladite sonde réactive étant capable de se fixer audit ligand cible et ayant une portion de queue polynucléotidique, ledit brin amplificateur comprenant un brin polynucléotidique capable de liaison à ladite queue polynucléotidique de ladite sonde réactive et ledit brin marqueur étant capable de liaison audit brin amplificateur et ayant un fragment marqueur susceptible de détecter ledit réactif pour former un réseau de brins polynucléotidiques fixé audit ligand cible.

13. Le nécessaire de la revendication 12 comprenant de plus un second brin amplificateur et un second brin marqueur, ledit second brin amplificateur étant capable de liaison audit premier brin amplificateur et ledit second brin marqueur étant capable de liaison audit second brin amplificateur.

14. Le nécessaire de la revendication 12 ou 13, dans lequel ladite sonde réactive comprend une portion de sonde polynucléotidique complémentaire dudit ligand cible.

15. Le nécessaire de l'une quelconque des revendications 12 à 14, dans lequel ladite portion de queue de ladite sonde réactive, ledit brin amplificateur et ledit brin marqueur comprennent des homopolymères.

16. Le nécessaire de l'une quelconque des revendications 12 à 15, dans lequel ledit brin amplificateur et ledit brin marqueur sont fixés entre eux dans un complexe amplificateur-marqueur.

17. Le nécessaire de l'une quelconque des revendications 12 à 16, dans lequel ledit fragment marqueur est non radioactif.

**Ansprüche**

1. Verfahren zum Untersuchen von Proben auf einen Targetliganden eines biologischen Paars umfassend die Schritte von:
In-Berührung-bringen der Probe mit dem Reagens unter Bindungsbedingungen, wobei das Reagens eine Reagensprobe, einen Verstärkerstrang und einen Markierungsstrang umfaßt, die Reagensprobe geeignet ist, sich an den Targetliganden zu binden und ein Polynucleotid-Schwanzteil hat, der Verstärkerstrang einen Polynucleotidstrang umfaßt, geeignet zum Binden an das Schwanzteil und an eine Vielzahl der Markierungsstränge und der Markierungsstrang einen Polynucleotidstrang mit einem nachweisbaren Markierungsrest umfaßt, wobei das Reagens zum Bilden eines Netzwerkes von Polynucleotidsträngen an den Targetliganden gebunden ist,
Entfernen des ungebundenen Reagens von der Probe, und Überwachen der Probe nach dem Markierungsrest.

2. Verfahren nach Anspruch 1, wobei das Reagens einen zweiten Verstärkerstrang und einen zweiten Markierungsstrang umfaßt, wobei der zweite Verstärkerstrang geeignet zum Binden an den ersten Verstärkerstrang ist und der zweite Markierungsstrang geeignet zum Binden an den zweiten Verstärkerstrang ist, und einen Markierungsrest umfaßt, wobei das Verfahren ferner den zusätzlichen Schritt vor der Überwachung und nach dem in-Berührung-bringen der Probe mit dem Reagens, dem Probensondenverstärkerstrang und Markierungsstrang, umfaßt, der das in-Berührung-bringen der Probe, dem zweiten Verstärkerstrang und dem Markierungsstrang unter Bindungsbedingungen umfaßt, um ein Netzwerk aus Polynucleotidsträngen zu bilden.

3. Verfahren nach Anspruch 1 oder 2, wobei der Verstärkerstrang geeignet ist, sich an eine Vielzahl von Markierungssträngen zu binden.

4. Verfahren nach einem der Ansprüche 1 bis 3, wobei mindestens einer von dem Verstärkerstrang und dem Sondenstrang Markierungsreste umfaßt.

5. Verfahren nach einem der Ansprüche 1 bis 4, wobei die Reagenssonde ein zu dem Targetliganden komplementäres Polynucleotidsondenteil einschließt.

6. Verfahren nach einem der Ansprüche 1 bis 5, wobei das Schwanzteil der Reagenssonde, der Verstärkerstrang, und der Markierungsstrang Homopolymere einschließen.

7. Verfahren nach einem der Ansprüche 1 bis 6, wobei die Reagensprobe zuerst mit der Reagenssonde in Berührung gebracht wird, der Targetligand sich an die Reagenssonde binden darf, um einen Ligandenrezeptorkomplex zu bilden, gefolgt von dem in-Berührung-bringen des Ligandenreagenskomplexes mit einem Reagensverstärkerstrang und Reagensmarkierungsstrang, wobei der Verstärkerstrang und Markierungsstrang miteinander verbunden sind, um einen Verstärkermarkierungskomplex zu bilden.

8. Verfahren nach einem der Ansprüche 1 bis 7, wobei der Markierungsrest nicht radioaktiv ist.

9. Reagenszusammensetzung zum Erhöhen der signalerzeugenden Fähigkeiten einer Reagenssonde mit einem Polynucleotid-Schwanzteil umfassend:
einen Polynucleotidverstärkerstrang und eine Vielzahl daran gebundener Markierungsstränge, wobei der Verstärkerstrang geeignet ist, sich an eine Reagenssonde zu binden, die Markierung einen wahrnehmbaren Markierungsrest hat, der Verstärkerstrang zum Binden an die Reagenssonde und der Markierungsstrang zum Binden an den Verstärkerstrang ein nachweisbares Netzwerk von Polynucleotidsträngen bilden.

10. Reagenszusammensetzung nach Anspruch 9, wobei mindestens einer des Verstärkerstranges und des Sondenstranges Markierungsreste umfaßt.

11. Reagenszusammenstellung nach Anspruch 9 oder 10, wobei der Verstärkerstrang und der Markierungsstrang komplementäre Homopolymere sind.

12. Ausrüstung zum Untersuchen einer Probe von Targetliganden eines biologischen Paares umfassend:
eine Reagens umfassend Reagenssonde, Verstärkerstrang und Markierungsstrang, wobei die Reagenssonde geeignet ist, sich an den Targetliganden zu binden und einen Polynucleotid-Schwanzteil hat, der Verstärkerstrang einen Polynucleotidstrang einschließt, geeignet zum Binden an den Polynucleotidschwanz der Reagenssonde, und der Markierungsstrang geeignet zum Binden an den Verstärkerstrang ist und einen Markierungsrest hat, geeignet zum Nachweisen des Reagens, um ein Netzwerk von Polynucleotidsträngen zu bilden, geeignet um die Verbindung mit dem Targetliganden nachzuweisen.

13. Ausrüstung nach Anspruch 12 umfassend ferner einen zweiten Verstärkerstrang und einen zweiten Markierungsstrang, wobei der zweite Verstärkerstrang geeignet ist, sich an den ersten Verstärkerstrang zu binden und der zweite Markierungsstrang geeignet ist, sich an den zweiten Verstärkerstrang zu binden.

14. Ausrüstung nach Anspruch 12 oder 13, wobei die Reagenssonde ein zu dem Targetliganden komplememtäres Polynucleotidsondenteil umfaßt.

15. Ausrüstung nach einem der Ansprüche 12 bis 14, wobei der Schwanzteil der Reagenssonde, der Verstärkerstrang und der Markierungsstrang Homopolymere umfassen.

16. Ausrüstung nach einem der Ansprüche 12 bis 15, wobei der Verstärkerstrang und der Markierungsstrang in einem Verstärkermarkierungskomplex miteinander verbunden sind.

17. Ausrüstung nach einem der Ansprüche 12 bis 16, wobei der Markierungsrest nicht radioaktiv ist.

FIG. 1

FIG. 2

FIG. 3

FIG. 4

FIG. 5

FIG. 6a

FIG. 6b

FIG. 7

FIG. 8